# EUROPÄISCHE PATENTSCHRIFT

(11) **EP 0 836 419 B1**
(45) Veröffentlichungstag und Bekanntmachung des Hinweises auf die Patenterteilung: **26.01.2000**
(21) Anmeldenummer: 96915940.9
(22) Anmeldetag: 17.06.1996
(51) Int. Cl.: A61B 7/04, A61B 5/0408, A61B 5/00

(54) **ELEKTRONISCHES STETHOSKOP**
ELECTRONIC STETHOSCOPE
STETHOSCOPE ELECTRONIQUE

(30) Priorität: 06.07.1995 CH 196895
(43) Veröffentlichungstag der Anmeldung: 22.04.1998
(73) Patentinhaber: CADItec AG, 6343 Rotkreuz (CH)
(72) Erfinder: Granzotto, Artemio, 8005 Zürich (CH)
(74) Vertreter: Spierenburg, Pieter
(86) Internationale Anmeldenummer: CH9600230
(87) Internationale Veröffentlichungsnummer: WO9701987

(56) Entgegenhaltungen:
- DE-A- 3 638 143
- DE-A- 3 804 616
- DE-A- 4 244 646
- DE-U- 1 952 893
- DE-U- 8 624 683
- DE-U- 8 911 113
- Biophysical Measurements -- Peter Strong -- Tektronix 1970

## Beschreibung

Die Erfindung betrifft ein beidohrig nutzbares, multifunktionales, vorzugsweise bügelarmiertes elektronisches Stethoskop gemäss dem Oberbegriff des Patentanspruchs 1.

Stethoskope sind Instrumente für die Untersuchung der Schallphänomene beziehungsweise zur Auskultation von Organtätigkeiten. Die heute üblichen Stethoskope sind beidohrig nutzbare, bügelarmierte Schlauchstethoskope, die als Kopfstück eine offene Glocke oder ein mittels einer Membran verschlossenes Teil aufweisen. Neben den mechanischen Schlauchstethoskopen sind auch elektronische Stethoskope auf dem Markt erhältlich. Unter dem Kopfstück eines Stethoskops versteht man den auf dem Körper des abzuhörenden Patienten auflegbaren Abhörtrichter. Der Abhörtrichter steht in Wirkverbindung mit einem Bruststück, von dem aus zwei Bügel zu den Ohrstücken führen.

Während beim Schlauchstethoskop die akustischen Signale vom objektseitigen Trichter über das Bruststück und die beiden Bügel direkt zu den Ohren des untersuchenden Arztes geführt werden, werden beim elektronischen Stethoskop die akustischen Signale durch ein im Kopfstück angeordnetes Mikrofon aufgenommen, in elektrische Signale gewandelt, zu den ohrseitigen Lautsprechern geleitet und dort verstärkt ausgesendet.

Neben den herkömmlichen elektronischen Stethoskopen sind auch Spezialmodelle bekannt für die Phonokardiographie. Ein phonokardiographisch arbeitendes Stethoskop ist beispielsweise aus der US-A-4840183 bekannt. Ein weiteres phonokardiographisch arbeitendes Stethoskop, welches mit entsprechenden Aufzeichnungseinheiten verbindbar ist, beschreibt die US-A-5025809.

Während bei den phonokardiographisch arbeitenden Stethoskopen, die durch die Herztätigkeit verursachten Herzgeräusche aufgenommen und verstärkt werden und gegebenfalls entsprechend aufgezeichnet werden, zeichnen die Elektrokardiographen das Kurvenbild in zeitlichem Verlauf der bioelektrischen Potentiale, die bei der Erregungsausbreitung und Erregungsrückbildung im Herz entstehen.

Die Ableitung der bioelektrischen Potentiale erfolgt mit Hilfe von auf der Körperoberfläche angebrachten Elektroden. Die im zeitlichen Ablauf dargestellten ermittelten Daten bilden ein Elektrokardiogramm (EKG), welches verschiedene diagnostisch auswertbare Faktoren aufzeigt.

Ferner ist aus der US-A-5394880 ein oesophagiales Stethoskop bekannt, das ein in die Speiseröhre einzuführendes Katheter aufweist, in dem ein kardiophonisch arbeitendes Mikrofon sowie mehere Sensoren angeordnet sind. Insbesondere ist hier ein Sensor zur Körpertemperaturmessung sowie zwei Elektroden für elektrokardiographische Aufzeichnungen vorgesehen. Die über das Katheter empfangenen Signale werden über eine elektrische Leitung nach aussen geführt zu einem Stecker, der die Verbindung zu entsprechenden Aufzeichnungsgeräten herstellt.

Während herkömmliche elektronische Stethoskope und die bekannten Elektrokardiographen Instrumente sind, die der Allgemeinpraktiker im täglichen Einsatz hat, sind oesophagiale Stethoskope hochsensible Geräte, die nur im klinischen Einsatz verwendet werden.

Aus DE-A-38 04 616 ist andererseits ein Stethoskop bekannt, bei welchem im Kopfstück fest angeordnete Elektroden vorgesehen sind, die EKG-Signale aufnehmen sollen, welche auf einem in den Schlauchleitungen vorgesehenen Display angezeigt werden können. Eine standardisierte EKG-Registrierung kann mit diesem Stethoskop nicht vorgenommen werden. Wegen der relativen Unhandlichkeit des beschriebenen Stethoskops ist es fraglich, ob dieses je auf dem Markt angeboten worden ist.

Ferner ist aus DE-U-89 11 113 ein medizinisches Handgerät mit einem Drehschalter und einer Flüssigkristallanzeige bekannt, das wie ein grösserer elektronischer Rechner ausgestaltet ist. Auf der Rückseite des Handgerätes sind drei Elektroden in einem Dreieck angeordnet. Auf der Vorderseite ist ein Mikrofon vorgesehen, das offensichtlich mit einem Kopfhörer verbunden werden kann. Dieses Gerät lässt sich kaum mit einem wesentlich praktischer zu handhabenden Stethoskop vergleichen.

Es ist nun Aufgabe der vorliegenden Erfindung, ein für den Allgemeinpraktiker mobil einsetzbares multifunktionales Stethoskop anzubieten, welches es erlaubt, neben dem Auskultieren gleichzeitig eine einfache elektrokardiographische Untersuchung vorzunehmen, bei welcher ein mehr oder weniger standardisiertes EKG aufgezeichnet wird.

Diese Aufgabe wird durch ein elektronisches Stethoskop mit den Merkmalen des Patentanspruchs 1 gelöst.

Eine möglichst platz- und gewichtssparende Lösung ergibt sich, wenn man die im Kopfstück angeordnete Displayanordnung in der Form einer Flüssigkristall-Anzeige gestaltet. Die preiswerteste Lösung ergibt sich, wenn man die drei Elektroden zur Erstellung des EKG fixiert innerhalb des Kopfstückes anordnet.

Fixiert man lediglich eine der drei Elektroden innerhalb des Kopfstückes, während man die beiden anderen Elektroden am Stethoskop-Kopfstück beweglich anordnet, so lässt sich dadurch das Einthoven'sche Dreieck verändern, beziehungsweise vergrössern. Die grösste Variationsmöglichkeit ergibt sich, wenn man alle drei der Erstellung des EKG dienenden Elektroden beweglich anordnet.

Eine besonders bevorzugte Ausführungsform sieht vor, dass man die beweglichen Elektroden in an der Peripherie der Auflagefläche des Stethoskopkopfes schwenkbar angeordneten Armen anordnet, die im nichtausgeschwenkten Zustand mindestens annähernd einen geschlossenen Auflagering bilden. Dies erlaubt mittels einem relativ kleinen Stethoskopkopf trotzdem ein relativ grosses Einthoven'sches Dreieck für die Elektroden zu bilden. Dies ergibt auch ein entsprechend aussagekräftiges EKG. Die letztgenannte Lösung lässt sich zusätzlich verbessern, indem man die schwenkbaren Arme so gestaltet, dass sie lediglich in der ausgeschwenkten Lage mit der Auswerteeinheit elektrisch in Verbindung stehen. Hiermit wird vermieden, dass wenig aussagekräftige Daten für die Erstellung eines EKG aufgezeichnet werden.

Das herkömmliche Stethoskop, seit ca. 100 Jahren bekannt, ist deshalb zum meist benutzten Diagonse-Instrument des Arztes geworden, weil es leicht, einfach handlich, ohne Vorbereitung anwendbar, immer in der Tasche oder um den Hals mitgetragen, zeitverzugslos eingesetzt werden kann. Alle übrigen Instrumente sind separate, meist elektrische Geräte, die Platz brauchen, aufgestellt werden müssen, usw..

Das erfindungsgemässe elektronische Stethoskop erweitert diese bisherige einfache (akustische) Diagnosemöglichkeit um die gleichzeitig, ebenso einfache Erfassung, visuelle Darstellung und Beurteilung der bioelektrischen Erregung der Herztätigkeit. Damit werden in der ersten ärztlichen Examination schon wichtige Information über Unregelmässigkeiten in der Herzfrequenz, über sogenannte Tachykariden, über Kammer-Flimmern und weitere, sonst erst später erkannte Probleme sofort erkannt.

Das erfindungsgemässe elektronische Stethoskop lässt sich zusätzlich mit Sensoren zur Blutdruck- und/oder Temperaturmessung ausrüsten. Die ebenfalls im Kopfstück untergebrachte Auswerteeinheit bringt normal die Signale direkt zur Aufzeichnung. Die Signale lassen sich aber auch in einen Datenspeicher eingeben und erst von dort zu einem stillstehenden Bild auf die Displayanordnung übertragen.

In der nachfolgenden Beschreibung ist ein Ausführungsbeispiel des Erfindungsgegenstandes anhand beiliegender Zeichnungen detailliert beschrieben.

Es zeigt:

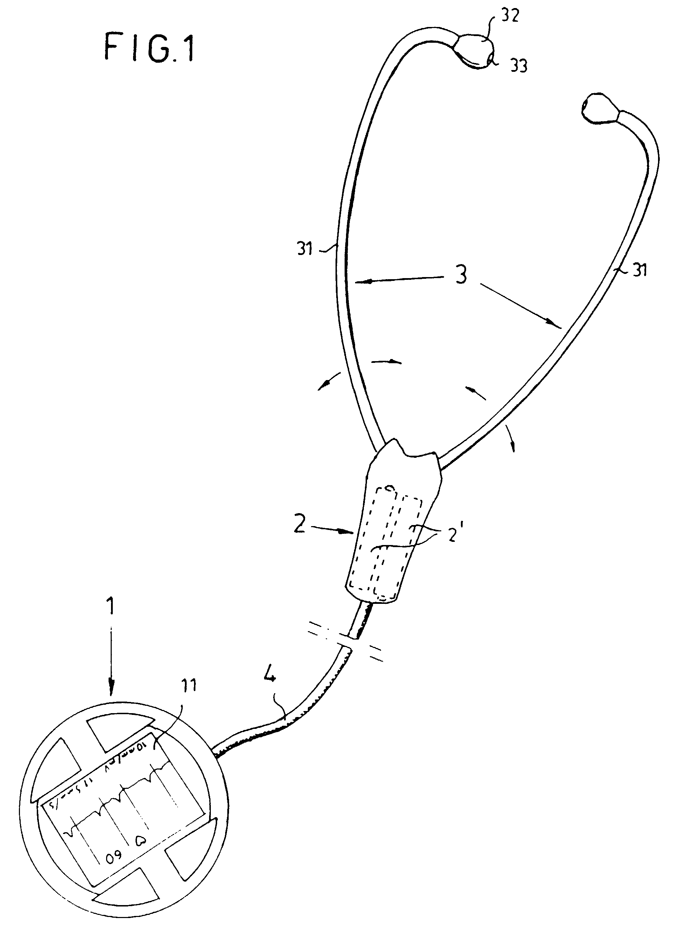

- Fig. 1: eine Gesamtansicht des elektronischen Stethoskops;
- Fig. 2: eine Seitenansicht des Kopfstückes desselben Stethoskops und
- Fig. 3: eine Aufsicht auf das Kopfstück des Stethoskops nach Fig. 2,
- Fig. 4: eine anlageseitige Aufsicht auf das Kopfstück des erfindungsgemässen elektronischen Stethoskops und
- Fig. 5: dieselbe Ansicht eine Variante des Stethoskopkopfes.
- Fig. 6: zeigt einen speziell gestalteten bogenförmigen Arm in der Aufsicht auf die Elektrode und
- Fig. 7: denselben Arm in der Seitenansicht.

Das erfindungsgemässe elektronische Stethoskop weist den üblichen Aufbau eines Schlauchstethoskopes auf. Es hat den auf den Patienten aufzulegenden Stethoskopkopf oder Kopfstück 1, welches über eine Verbindungsleitung 4 mit dem sogenannten Bruststück in Verbindung steht. An das Bruststück 2 schliessen die beiden Ohrstücke 3 an.

Im Bruststück 2, welches als Kunststoffgehäuse ausgebildet ist, ist die Speisequelle 2' untergebracht. Die Speisequelle besteht aus einer oder mehreren Batterien beziehungsweise Akkus, die vorzugsweise nachgeladen werden können. An das Bruststück 2 schliessen die beiden Ohrstücke 3 an. Diese umfassen je einen Bügel 31, die gelenkig mit dem Bruststück 2 verbunden sind. Endständig sind an den Bügeln 31 je eine sogenannte Olive 32 aufgesteckt, die jeweils eine Schallöffnung 33 aufweisen. In den Oliven 32 ist jeweils ein Lautsprecher untergebracht.

Die erfindungsgemässe Ausgestaltung des elektronischen Stethoskopes beschränkt sich im wesentlichen auf den Aufbau des Stethoskopes 1. Das wesentlichste Merkmal dieses Stethoskopkopfes besteht in der integrierten Displayanordnung 11. Die Displayanordnung 11 ist hier als Flüssigkristall-Anzeige ausgebildet. Die Display-Anordnung 11 hat verschiedene Anzeigefelder 12, die über Druck auf die Betätigungstasten 13 aktiviert werden können. Die Anzeigefelder 12 können beispielsweise die Herzfrequenz, die Körpertemperatur oder ein EKG aufzeigen. In den entsprechenden Feldern 12 werden auch die Massstabsangaben zum Auswerten des aufgezeigten EKG angezeigt. Aus der hier ersichtlichen Angabe ersieht man, dass die Höhe eines Piks von 10 mm einem mV entspricht. Der Verlauf auf der X-Achse ist ebenfalls angegeben. Im dargestellten Beispiel entsprechen 12,5 mm einer Sekunde.

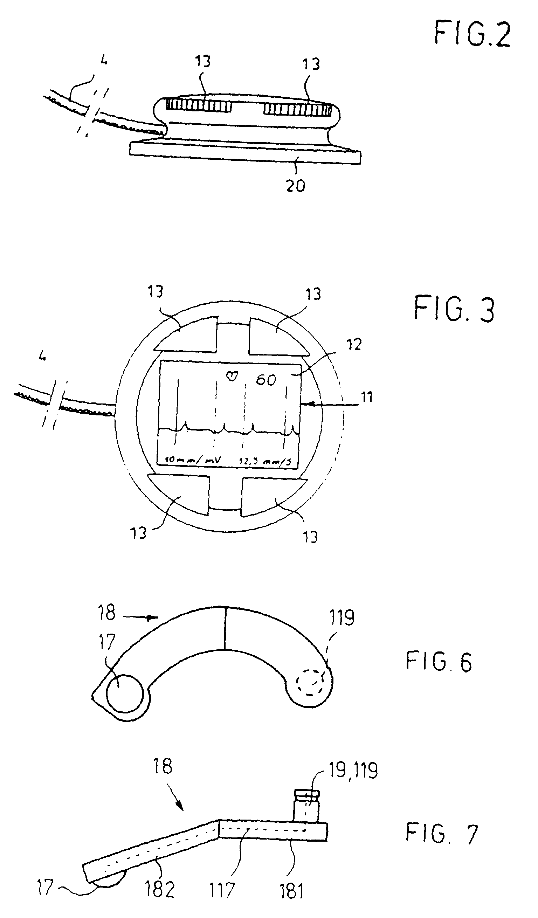

In der Seitenansicht nach Fig. 2 ist auch ein Auflagering 20 ersichtlich, der als Auflagefläche auf den Patienten dient.

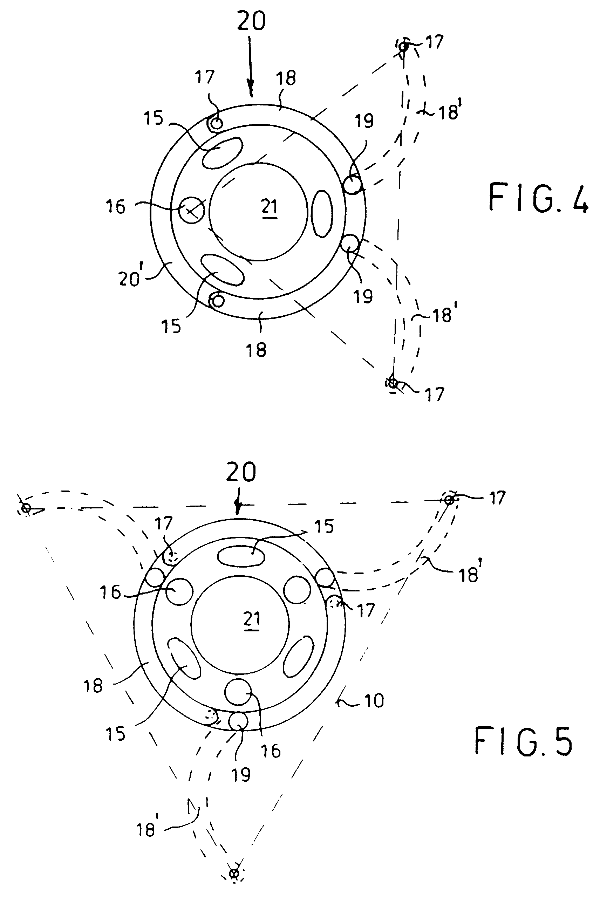

Betrachtet man die Ansicht der Auflagefläche, wie dies die Figuren 4 und 5 zeigen, so erkennt man die funktionalen Teile. In der Mitte des Stethoskopkopfes 1 ist das Mikrofon 21 angeordnet. Dieses ist gegenüber der Auflagefläche, welche durch den Auflagering 20 gebildet ist, nach innen zurückversetzt, so dass das Mikrofon nicht direkt auf der Haut aufliegt. Es ist jedoch möglich, dieses Mikrofon wie bei bekannten elektronischen Stethoskopen mit einer Membran zu versehen, die dann mindestens annähernd auf dem Niveau des Auflageringes 20 liegt und somit auf der Körperoberfläche des Patienten zum Anliegen kommt. Der Auflagering 20 ist üblicherweise einige Millimeter dick, so dass dieser einen Resonanzhohlraum begrenzt. Der Auflagering ist in der Ausführung gemäss der Fig. 4 aus drei Teilen zusammengesetzt. Rund ein Drittel des Auflageringes 20 besteht aus einem fest am Stethoskopkopf 1 angeordneten Ringteil 20', während die beiden anderen Auflageringteile als bogenförmige Arme 18 ausgestaltet sind, die über Drehgelenke 19 nach aussen geschwenkt werden können.

Innerhalb des Stethoskopkopfes ist eine Elektrode 16 fest angeordnet, während die beiden anderen Elektroden, die der Erstellung eines EKG dienen, an den Armen 18 endständig befestigt sind. Neben den drei Elektroden 16, 17 sind am Stethoskopkopf 1 zusätzlich drei Sensoren 15 angebracht. Diese Sensoren 15 liegen etwa in der Ebene der Oberfläche des Auflageringes 20. Diese Sensoren können dazu verwendet werden, andere, die Körperfunktion wiedergebende Merkmale zu ermitteln. So können ein oder mehrere Sensoren zur Bestimmung der Körpertemperatur dienen oder eine oder mehrere Sensoren der Ermittlung des Blutdruckes dienen.

Ein Stethoskopkopf gemäss der Fig. 4 ist folglich multifunktionell. Solange die Arme 18 nicht ausgeschwenkt sind, bilden sie zusammen mit dem Auflageringteil 20' den Auflagering 20 und umschliessen somit einen Resonanzraum, welcher der Auskultierung verschiedener Körperfunktionen dient. In dieser Lage der Arme 18 kann das Mikrofon 21 auch zur Aufzeichnung eines Phonokardiogrammes dienen. Hingegen ist es erforderlich, die Arme 18 in die Lage 18' zu schwenken, um ein Elektrokardiogramm aufzuzeichnen. In der ausgeschwenkten Lage bilden die fixierte Elektrode 16 und die beweglichen Elektroden 17 an den ausgeschwenkten Armen 18' ein sogenanntes Einthoven'sches Dreieck 10.

Um Fehlanzeigen zu vermeiden, kann es von Vorteil sein, wenn die Drehgelenke 19 mit Schaltkontakten in Verbindung stehen, die bewirken, dass erst eine elektrische Verbindung der Elektroden 17 mit der Auswerteeinheit gebildet ist, wenn die Arme 18 in die vorschriftsgemäss vollständig ausgeschwenkte Lage 18' gedreht sind.

In der Ausführung gemäss der Fig. 5 ist der Stethoskopkopf insofern modifiziert, dass einerseits drei fixierte Elektroden 16 angebracht sind und ferner der Auflagering 20 aus drei beweglichen Armen gebildet ist. Auch hier ist jeweils am Drehgelenk 19 abgelegenen Ende jedes Armes 18 eine bewegliche Elektrode 17 angeordnet. Hier schaltet man die Elektroden 16 beziehungsweise 17 so, dass im nichtausgeschwenkten Zustand der Arme 18 nur die fest angeordneten Elektroden 16 funktionieren, während nach Ausschwenkung der Arme 18 in die Lage 18' nur die beweglichen Elektroden 17 arbeiten. Diese Anordnung ergibt ein wesentlich vergrössertes Einthoven'sches Dreieck 10.

Um einen gesicherten Auflagekontakt der an den Armen 18 angebrachten Elektroden 17 auf den Patienten zu erreichen, ist es vorteilhaft, diese Arme 18 flexibel zu gestalten. Eine solche Ausführungsform ist in den Figuren 6 und 7 dargestellt. Prinzipiell könnten die Arme zweiteilig und gelenkig gefertigt werden, doch ergibt dies Probleme bei der Desinfektion des Gerätes. Statt dessen wurde der Arm 18 einstückig aus zwei verschiedenen Kunststoffen in zwei Stufen gespritzt und die elektrischen Anteile, nämlich der Sensor 17 und der Steckkontakt 119, der zugleich das Drehglenk 19 bildet, zusammen mit der leitenden Verbindung 117 eingebettet. Hierbei wählt man für den Armteil 181, an dem das Drehgelenk angreift, einen harten Kunststoff und für den anderen Armteil 182, an dem der Sensor 17 angeordnet ist, einen weichen Kunststoff. Die beiden Armteile 181, 182 schliessen dabei einen stumpfen Winkel ein, der sich jedoch unter Druck auf den Stethoskopkopf verändert.

Die für ein solches Stethoskop erforderlichen elektronischen Bauteile sind auf dem Markt erhältlich. Ihre Programmierung muss auf die Anwendung optimiert werden.

Die entsprechende Elektronik umfasst im wesentlichen einen Digital-Analog-Wandler, Verstärker, Taktgeber für die Zeitmessung und vorzugsweise eine Speichereinheit, in der gemessene Resultate zwischengespeichert werden können und für die Anzeige auf der Displayanordnung abrufbar sind. Entsprechend kann ein stillstehendes EKG-Bild aufgezeigt werden, welches für die Diagnose genauer betrachtet werden kann.

## Patentansprüche

1. Multifunktionales, vorzugsweise bügelarmiertes, elektronisches Stethoskop mit mehreren im Kopfstück (1) um ein Mikrophon (21) angeordneten Sensoren und/oder Elektroden (15, 16, 17), mit in den Ohrstücken (3) angebrachten Lautsprechern, und mit einer Displayanordnung zur visuellen Darstellung der von den Sensoren bzw. Elektroden ermittelten Körperfunktionswerte, die von einer Auswerteeinheit erfasst und für die Displayanordnung aufbereitet sind, dadurch gekennzeichnet, dass mindestens drei der Sensoren bzw. Elektroden mindestens annähernd in Anordnung eines Einthoven'schen Dreiecks befestigte und/oder bringbare Elektroden (16, 17) zur Erfassung eines Elektrokardiogrammes (EKG) sind, und die Displayanordnung zur Visualisierung des EKG im Kopfstück untergebracht ist.

2. Elektronisches Stethoskop nach Anspruch 1, dadurch gekennzeichnet, dass die Displayanordnung (11) eine Flüssigkristallanzeige ist.

3. Elektronisches Stethoskop nach Anspruch 1, dadurch gekennzeichnet, dass die drei Elektroden (16) zur Erstellung eines EKG fix innerhalb des Kopfstückes (1) angeordnet sind.

4. Elektronisches Stethoskop nach Anspruch 1, dadurch gekennzeichnet, dass mindestens zwei der drei Elektroden (16, 17) zur Erfassung des EKG am Kopfstück (1) beweglich angeordnet sind.

5. Elektronisches Stethoskop nach Anspruch 4, dadurch gekennzeichnet, dass die beweglichen Elektroden (17) schwenkbar am Kopfstück (1) angeordnet sind und im ausgeschwenkten Zustand die drei Elektroden (17) mindestens annähernd ein Einthoven'sches Dreieck bilden.

6. Elektronisches Stethoskop nach Anspruch 4 oder 5, dadurch gekennzeichnet, dass eine der Elektroden (16) fix innerhalb des Kopfstückes (1) angeordnet ist.

7. Elektronisches Stethoskop nach Anspruch 4 oder 5, dadurch gekennzeichnet, dass drei bewegliche Elektroden (17) und drei Elektroden (16) fix innerhalb des Kopfstückes (1) angeordnet sind.

8. Elektronisches Stethoskop nach Anspruch 5, dadurch gekennzeichnet, dass die beweglichen Elektroden (17) in an der Peripherie der Auflageflläche des Stethoskopkopfes (1) schwenkbar angeordneten Armen (18, 18') angeordnet sind , die im nicht ausgeschwenkten Zustand mindestens annähernd einen Auflagering bilden.

9. Elektronisches Stethoskop nach Anspruch 5, dadurch gekennzeichnet, dass die beweglichen Elektroden (17) in schwenkbaren Armen (18, 18') angeordnet sind, die lediglich in der ausgeschwenkten Lage (18') mit der Auswerteeinheit elektrisch in Verbindung stehen.

10. Elektronisches Stethoskop nach Anspruch 1, dadurch gekennzeichnet, dass im Kopfstück (1) zusätzlich mindestens ein Sensor (15) zur Blutdruckmessung und/oder Temperaturmessung vorgesehen ist.

11. Elektronisches Stethoskop nach Anspruch 1, dadurch gekennzeichnet, dass die Auswerteeinheit mit mindestens einem Datenspeicher in Wirkverbindung steht, der intern oder extern abrufbar ist.

12. Elektronisches Stethoskop nach Anspruch 11, dadurch gekennzeichnet, dass der Datenspeicher mit einem Sender im Wirkverbindung steht.

13. Elektronisches Stethoskop nach Anspruch 8, dadurch gekennzeichnet, dass die schwenkbaren Arme (18) flexibel geformt sind.

14. Elektronisches Stethoskop nach Anspruch 13, dadurch gekennzeichnet, dass die flexiblen Arme (18) aus zwei integral miteinander verbundenen Armteilen (181, 182) gestaltet sind, die aus Kunststoffen mit unterschiedlicher Biegungselastizität gefertigt sind.

## Claims

1. Multifunctional electronic stethoscope preferably fitted with a bow, with several sensors and/or electrodes (15, 16 17) arranged around a microphone (21) in the headpiece (1) and with loudspeakers mounted in the earpieces (3), and having a display arrangement for the visual representation of the body function values captured by the sensors and/or electrodes, which values are collected by an evaluation unit and processed for the display arrangement, characterised in that at least three of the sensors and/or electrodes are electrodes (16, 17) for obtaining an electrocardiogram (ECG), which electrodes are fixed and/or movable into at least approximately the arrangement of an Einthoven Triangle, and in that the display arrangement for visualisation of the ECG is mounted in the headpiece.

2. Electronic stethoscope according to Claim 1, characterised in that the display arrangement (11) is a liquid-crystal display.

3. Electronic stethoscope according to Claim 1, characterised in that the three electrodes (16) for obtaining the ECG are in a fixed arrangement inside the headpiece (1).

4. Electronic stethoscope according to Claim 1, characterised in that at least two of the three electrodes (16, 17) for obtaining the ECG are mounted movably on the headpiece (1).

5. Electronic stethoscope according to Claim 4, characterised in that the movable electrodes (17) are mounted pivotably to the headpiece (1) and the three electrodes (17) form at least approximately an Einthoven Triangle in the swivelled-out position.

6. Electronic stethoscope according to Claim 4 or 5, characterised in that one of the electrodes (16) is mounted in a fixed position inside the headpiece (1).

7. Electronic stethoscope according to Claim 4 or 5, characterised in that three movable electrodes (17) and three electrodes (16) in a fixed position inside the headpiece (1) are arranged.

8. Electronic stethoscope according to Claim 5, characterised in that the movable electrodes (17) are mounted in arms (18, 18') attached pivotably to the periphery of the contact surface of the stethoscope head (1), which arms form at least approximately a contact ring when in the non-swivelled-out position.

9. Electronic stethoscope according to Claim 5, characterised in that the movable electrodes (17) are mounted in pivotable arms (18, 18') which are electrically connected to the evaluation unit only when in the swivelled-out position (18').

10. Electronic stethoscope according to Claim 1, characterised in that the headpiece (1) is additionally provided with at least one sensor (15) for measurement of blood pressure and/or temperature.

11. Electronic stethoscope according to Claim 1, characterised in that the evaluation unit is effectively connected to at least one data memory from which data can be called up either internally or externally.

12. Electronic stethoscope according to Claim 11, characterised in that the data memory is effectively connected to a transmitter.

13. Electronic stethoscope according to Claim 8, characterised in that the pivotable arms (18) are formed flexibly.

14. Electronic stethoscope according to Claim 13, characterised in that the flexible arms (18) are formed of two integrally connected arm sectors (181, 182) which are made of plastics with different flectional elasticity.

## Revendications

1. Stéthoscope électronique, multifonctionnei, de préférence à branches en arceau, comprenant plusieurs capteurs et/ou électrodes (15, 16, 17) disposés autour d'un microphone (21), des haut-parleurs disposés dans les pièces auriculaires (3) et un dispositif d'affichage pour la représentation visuelle des paramètres fonctionnels du corps captés par les capteurs ou électrodes, qui sont saisis par une unité d'exploitation et préparés pour le dispositif d'affichage, caractérisé en ce qu'au moins trois des capteurs ou électrodes sont des électrodes (16, 17) destinées à saisir un électrocardiogramme (ECG) qui sont fixés et/ou peuvent être placés au moins approximativement dans la disposition d'un triangle de Einthoven, et le dispositif d'affichage est logé dans la pièce de tête pour la visualisation de l'ECG.

2. Stéthoscope électronique selon la revendication 1, caractérisé en ce que le dispositif d'affichage (11) est un affichage à cristaux liquides.

3. Stéthoscope électronique selon la revendication 1, caractérisé en ce que les trois électrodes (16) destinées à la production d'un ECG sont disposées en position fixe à l'intérieur de la pièce de tête (1).

4. Stéthoscope électronique selon la revendication 1, caractérisé en ce qu'au moins deux des trois électrodes (16, 17) destinées à la production d'un ECG sont disposées mobiles sur la pièce de tête (1) pour la prise de l'ECG.

5. Stéthoscope électronique selon la revendication 4, caractérisé en ce que les électrodes mobiles (17) sont disposées oscillantes sur la pièce de tête (1) et que, dans l'état écarté, les trois électrodes (17) forment au moins approximativement un triangle de Einthoven.

6. Stéthoscope électronique selon la revendication 4 ou 5, caractérisé en ce qu'une des électrodes (16) est disposée en position fixe à l'intérieur de la pièce de tête (1).

7. Stéthoscope électronique selon la revendication 4 ou 5, caractérisé en ce que trois électrodes mobiles (17) et trois électrodes (6) placées en position fixe à l'intérieur de la pièce de tête (1) sont disposées.

8. Stéthoscope électronique selon la revendication 5, caractérisé en ce que les électrodes mobiles (17) sont disposées dans des bras (18, 18') montés pivotants à la périphérie de la surface de portée de la tête (1) du stéthoscope et qui, dans l'état non écarté par pivotement, forment au moins approximativement un anneau de portée.

9. Stéthoscope électronique selon la revendication 5, caractérisé en ce que les electrodes mobiles (17) sont montées dans des bras pivotants (18, 18) qui ne sont en communication électrique avec l'unité d'exploitation que dans la position écartée (18').

10. Stéthoscope électronique selon la revendication 1, caractérisé en ce que, dans la pièce de tête (1) est disposé en supplément au moins un capteur (15) pour la mesure de la pression sanguine et/ou pour la mesure de la température.

11. Stéthoscope électronique selon la revendication 1, caractérisé en ce que l'unité d'exploitation est en liaison effective avec au moins une mémoire de données qui peut être appelée de l'intérieur ou de l'extérieur.

12. Stéthoscope électronique selon la revendication 11, caractérisé en ce que la mémoire de données est en liaison effective avec un émetteur.

13. Stéthoscope électronique selon la revendication 8, caractérisé en ce que les bras pivotants (18) sont flexibles.

14. Stéthoscope électronique selon la revendication 13, caractérisé en ce que les bras flexibles (18) sont formés de deux parties de bras (181, 182) assemblées l'une à l'autre en une seule pièce, qui sont fabriquées en matières plastiques possédant des élasticités de flexion différentes.
